Europäisches Patentamt

European Patent Office

Office européen des brevets

(19)

(11) Veröffentlichungsnummer: **0 321 588**
**A1**

(12) # EUROPÄISCHE PATENTANMELDUNG

veröffentlicht nach Art. 158 Abs. 3 EPÜ

(21) Anmeldenummer: 88906229.5

(22) Anmeldetag: 23.06.88

(51) Int. Cl.⁴: **A 61 F 2/24**

Daten der zugrundeliegenden internationalen Anmeldung:

(86) Internationale Anmeldenummer:
PCT/SU 88/00137

(87) Internationale Veröffentlichungsnummer:
WO 88/10105 (29.12.88 88/28)

(30) Priorität: 25.06.87 SU 4261093

(43) Veröffentlichungstag der Anmeldung: 28.06.89
Patentblatt 89/26

(84) Benannte Vertragsstaaten: BE DE GB IT LU NL SE

(71) Anmelder: BUKATOV, Alexandr Semenovich, pl.
Pobedy, 1-A-122, Moscow, 121293 (SU)
Anmelder: IOFIS, Naum Abramovich, Lomonosovsky
pr. 23-416, Moscow, 117311 (SU)
Anmelder: EGOROV, Jury Grigorievich, Sevastopolsky
pr. 83-1-28, Moscow, 113461 (SU)

(71) Anmelder: DOBROVA, Natalya Borisovna, ul.
Matrosskaya tishina 17-26, Moscow, 107076 (SU)
Anmelder: KOSTRETSOV, Anatoly Stepanovich, ul.
Bakuninskaya, 10/12-7, Moscow, 107005 (SU)
Anmelder: AGAFONOV, Andrei Vasilievich, ul.
Smolnaya 63-149, Moscow, 125445 (SU)

(72) Erfinder: BUKATOV, Alexandr Semenovich, pl.
Pobedy, 1-A-122, Moscow, 121293 (SU)
Erfinder: IOFIS, Naum Abramovich, Lomonosovsky
pr. 23-416, Moscow, 117311 (SU)
Erfinder: EGOROV, Jury Grigorievich, Sevastopolsky
pr. 83-1-28, Moscow, 113461 (SU)
Erfinder: DOBROVA, Natalya Borisovna, ul. Matrosskaya
tishina 17-26, Moscow, 107076 (SU)
Erfinder: KOSTRETSOV, Anatoly Stepanovich, ul.
Bakuninskaya, 10/12-7, Moscow, 107005 (SU)
Erfinder: AGAFONOV, Andrei Vasilievich, ul.
Smolnaya 63-149, Moscow, 125445 (SU)

(74) Vertreter: Nix, Frank Arnold, Dr., Kröckelbergstrasse 15,
D-6200 Wiesbaden (DE)

(54) HERZKLAPPENPROTHESE.

(57) A heart valve prosthesis comprises a valve ring (1) with an
aperture (2) for passage of the blood. A disk (3) is mounted in
said aperture (2) for closing and opening the aperture. The disk
(3) is provided, on its upper or distal side (6), with an annular
curved groove (9) reducing the mass of the disk (3). The disk
(3) is floatably mounted between limiters (4, 5) of its movement.

EP 0 321 588 A1

## HERZKLAPPENPROTHESE
### Technisches Gebiet

Die vorliegende Erfindung bezieht sich auf das Gebiet Medizin, insbesondere die Kardiochirurgie, und zwar eine Herzklappenprothese, die zum Einsetzen anstelle einer beschädigten natürlichen Herzklappe bestimmt ist.

### Zugrundeliegender Stand der Technik

In letzten Jahren finden sogenannte Scheibenprothesen der Herzklappe immer mehr eine weitgehende Anwendung, die einen Klappenring bzw. -körper mit einer Öffnung zum Blutdurchfluß einschließen, in der eine Scheibenklappe eingesetzt wird, die zum Verschließen bzw. Zumachen und zum Öffnen der Innenöffnung während des Betriebs der Herzklappe vorgesehen ist. Die erwähnte Scheibeklappe wird vermittels einer oberen Stütze und unteren Abstützungsanordnung bzw. der sogenannten Hubbegrenzer schwimmend angeordnet. In dieser Herzklappenprothese weist die Scheibenklappe eine obere bzw. Distalseite und eine untere bzw. Proximalseite auf, von denen die Distalseite eine konvexe ist und eine Vertiefung für den oberen Hubbegrenzer besitzt, während die andere, die Proximalseite in Übereinstimmung mit der Konfiguration des unteren Hubbegrenzers ausgeführt ist, wie sie beispielsweise die Patentschrift der USA Nr. 4 057 857, herausgegeben für Fettel et al, oder Patentschrift der USA Nr. 4 713 071 herausgegeben für Iofis et al, wiedergibt.

Diese Herzklappenprothesen weisen jeweilige Vorteile auf, die bespielsweise darin bestehen, daß eine oberflächliche Berührung der Scheibenklappe in deren zuschließender Stellung mit den Abstützungsteilen der Hubbegrenzer erreicht wird, wobei nach der letztgenannten Patentschrift der zwei genannten, und zwar nach der Patentschrift der USA Nr. 4 713 071 diese oberflächliche Berührung sowohl in der zuschließenden als auch in der geöffneten Stellung der Klappenscheibe erreicht wird. Dabei weist diese Herzklappenprothesenart

- 2 -

eine ziemlich massive Scheibenklappe auf, und zwar dadurch, daß die Distalseite der Scheibenklappe unter einer entsprechenden Vergrößerung deren Rauminhaltes und demzufolge auch der Masse der Scheibenklappe der Herzklappenprothese konvex ausgeführt ist. In diesem Zusammenhang werden auch die Verhältnisse einer Winkelverschwenkung der Scheibenklappe und somit auch der Rehabilitation des Patienten nach der Operation ihrerseits verschlechtert.

Offenbarung der Erfindung

Der Erfindung liegt die Aufgabe zugrunde, die Scheibenklappe der Herzklappenprothese derart auszuführen, daß deren Masse verkleinert wird, wodurch die Verhältnisse sowohl einer Winkelverschwenkung der Scheibenklappe unter Sicherung der Verlängerung der Lebensdauer und Steigerung der Arbeitszuverlässigkeit der Klappenprothese als auch der Rehabilitation des Patienten verbessert werden.

Die gestellte Aufgabe wird dadurch gelöst, daß in der Herzklappenprothese, die einen Klappenring mit einer Öffnung zum Blutdurchfluß und eine in dieser Innenöffnung eingesetzte schwimmende Scheibenklappe einschließt, die eine Distal- und Proximalseite besitzt, die mit den Hubbegrenzern in Wechselwirkung treten, erfindungsgemäß die Distalseite der Scheibenklappe eine zwischen dem Mittelteil der Distalseite und dem Umfangsteil der Scheibenklappe liegende konkave Ringfläche besitzt, welche auf der ganzen Länge ihrer Kreislinie in diesen Umfangsteil glatt übergeht.

Das Vorliegen der erwähnten konkaven Ringfläche macht es möglich, die Masse der Scheibenklappe bedeutend herabzusetzen, was bei einer starken Schwächung der Tätigkeit des Herzmuskels von besonderer Wichtigkeit ist. Obwohl dabei die Winkelverschwenkung der Scheibenklappe wie in der bekannten Ausführung durch die entsprechende Ausbildung der Abstützungsteile zustandekommt, werden die Verhältnisse dieser Winkelverschwenkung durch die erwähnte Massenverkleinerung verbessert, da wie bekannt je kleiner die Masse der

- 3 -

Scheibenklappe ist, desto leichter verläuft deren Winkelverschwenkung.

In einer der Ausführungsvarianten der Erfindung wird es vorgesehen, daß die erwähnte konkave Ringfläche an der Distal- und Proximalseite einen gleichgroßen Krümmungsradius aufweist. In diesem Fall werden die besten hämodynamischen Bedingungen für den Durchfluß des Blutes durch die Innenöffnung des Klappenringes erreicht.

### Kurzbeschreibung der Zeichnungen

Nachstehend wird die Erfindung anhand der Beschreibung konkreter, die vorliegende Erfindung nicht einschränkender Ausführungsbeispiele und der beiliegenden Zeichnungen näher erläutert; in diesen zeigt:

Fig. 1 axonometrische, teilweise geschnittene Darstellung der Herzklappenprothese gemäß einer Ausführungsversion der vorliegenden Erfindung;

Fig. 2 geschnittene Einzelheit der Scheibenklappe aus der Fig. 1.

### Beste Ausführungsvariante der Erfindung

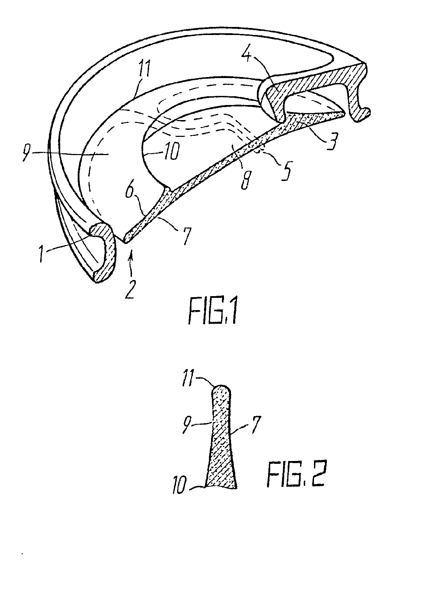

Zunächst betrachten wir Fig. 1, in der die Ausführung der Herzklappenprothese in Ganzen der Erfindung gemäß am besten zu ersehen ist. Wie aus der Fig. 1 zu ersehen ist, enthält die Herzklappenprothese einen Klappenring bzw. -körper 1, der in Form eines Zylinders mit Bünden ausgeführt ist, die zur Befestigung einer aus den Zeichnungen nicht ersichtlichen, um deren Überlastung durch überflüssige, in keiner Beziehung zu der Erfindung stehende Einzelheiten zu vermeiden, Manschette vorgesehen sind. Die Ausführung, Gestaltung und der Stoff einer solchen Manschette sind den auf dem vorliegenden Gebiet erfahrenen Fachleuten gut bekannt. Die Manschette dient der Befestigung der Herzklappenprothese selbst bei deren Einsetzen an den Umgebungsgeweben. Der Klappenring 1 umgibt die Klappeninnenöffnung 2 und bildet noch den Sitz für die Scheibenklappe 3. Diese Scheibenklappe 3 ist schwimmend eingesetzt, ähnlich wie sie in der in der vorliegenden Beschreibung als Druckschriftenhin-

- 4 -

weis erwähnten US-PS Nr. 4 713 071 ausführlich beschrieben ist, und in diesem Zustand vermittels der Hubbegrenzer in Form einer oberen Stütze 4 und unteren Abstützungsanordnung 5 zurückgehalten, von der nur eine Hälfte in Fig. 1 wiedergegeben ist.

Die Scheibenklappe 3 weist eine obere bzw. Distalseite 6 und untere bzw. Proximalseite 7 auf. Die Proximalseite 7 ist leicht konkav ausgeführt, während deren Krümmungsradius in Übereinstimmung mit der Konfiguration der unteren Abstützungsanordnung 5 gewählt ist. Im Mittelteil der Distalseite 6 ist eine Vertiefung 8 zur Aufnahme der oberen Stütze 4 ausgespart.

Die Ringfläche 9 an der oberen Distalseite 6 der Scheibenklappe 3 von den Rändern 10 der Vertiefung 8 aus zu dem Umfangskreis 11 der Scheibenklappe 3 hin wird auf der ganzen Länge des Kreises der Scheibenklappe 3 konkav ausgeführt. Der Krümmungsradius dieser konkaven Ringfläche 9 ist in der beschriebenen konkreten Version der Prothesenausführung gleich dem der Proximalseite 7 ausgeführt, wie aus der Fig. 2 gut zu ersehen ist. Selbstverständlich kann der Krümmungsradius der konkaven Ringfläche 9 sich von dem der Proximalseite 7 unterscheiden. Im allgemeinen ist die Krümmung der konkaven Ringfläche 9 so groß zu wählen, daß dadurch ein glatter Übergang zu dem Umfangskreis 11 erreicht wird, die geometrischen Einflußgrößen für den Blutdurchfluß bei der geöffneten Stellung der Scheibenklappe 3 sich nicht verschlechtern, d.h., daß keine Steigerung beispielsweise des hydraulischen Widerstandes auftritt.

Die obenbeschriebene Gestaltung der Distalseite 6, bei der auf der ganzen Länge des Scheibenkreises die konkave Ringfläche 9 vorhanden ist ., ermöglicht eine Verkleinerung der Masse der Scheibenklappe ohne Zerstörung deren kraftschlüssiger Verbindung mit den Hubbegrenzern und ohne Verschlechterung der physiologischen Eigenschaften der Herzklappenprothese, wobei bei Benutzung der Scheibenklappe der symmetrischen, doppelkon-

- 5 -

kaven, in der Fig. 2 wiedergegebenen Profilform der hydraulische Widerstand sich im Vergleich mit den bekannten Herzklappenprothesen sogar verringert. In diesem Zusammenhang sind die Hubbegrenzer bei so einer Gestaltung der Scheibenklappe 3 zweckmäßigerweise wie in der US-PS Nr. 4 713 071 beschrieben auszuführen, damit die Winkelverschwenkung der Scheibenklappe 3 zustandekommt. Dabei muß darauf aufmerksam gemacht werden, daß die Herabsetzung der Masse der Scheibenklappe jeweilige Verbesserungen bei den Verhältnissen der Winkelverschwenkung der Scheibenklappe beim Blutdurchfluß mit sich bringt.

Es wird die Arbeitsweise der beschriebenen Herzklappenprothese nicht erläutert, weil sie sich von der in der früher erwähnten US-PS Nr. 4 713 071 beschriebenen in nichts unterscheidet. Es muß nur darauf aufmerksam gemacht werden, daß die Anwendung der vorliegenden Erfindung ermöglicht, die Masse der Scheibenklappe etwa um 15% inbezug auf die bekannten Ausführungen herabzusetzen, ohne daß dabei die hämodynamischen Kennwerte der Herzklappenprothese gestört werden.

Industrielle Anwendbarkeit

Die Herzklappenprothese wird zum Ersetzen der Aorten- bzw. Mitralklappe dank der herabgesetzten Masse der Scheibenklappe weitgehend ausgenutzt, besonders wenn die Tätigkeit des Herzmuskels stark abgeschwächt ist. Die erfindungsgemäße Herzklappenprothese kann aus biologisch inerten Stoffen wie beispielsweise Titanlegierungen oder Kohlenstoffmaterialien hergestellt werden.

– 6 –

PATENTANSPRÜCHE:

1. Herzklappenprothese, die einen Klappenring (1) mit einer Öffnung zum Blutdurchfluß und eine in dieser Innenöffnung eingesetzte schwimmende Scheibenklappe (3) einschließt, die eine mit Hubbegrenzern (4, 5) in Wechselwirkung tretende Distal- und Proximalseite (6) und (7) aufweist, d a d u r c h  g e - k e n n z e i c h n e t, daß die Distalseite (6) eine konkave Ringfläche (9) besitzt, die zwischen dem Mittelteil dieser Distalseite und dem Umfangskreis (11) der Scheibenklappe (3) liegt und in diesen Umfangskreis auf dessen ganzer Länge glatt übergeht.

2. Herzklappenprothese nach Anspruch 1, d a d u r c h  g e k e n n z e i c h n e t, daß die konkave Ringfläche (9) der Distalseite und die Proximalseite selbst einen gleichgroßen Krümmungsradius besitzen.

- 7 -

# HERZKLAPPENPROTHESE

## ZUSAMMENFASSUNG

Die Herzklappenprothese enthält einen Klappenring (1) mit einer Öffnung (2)             . zum Blutdurchfluß. In dieser Innenöffnung (2) ist eine Scheibenklappe (3) zum Schließen und Öffnen der erwähnten Innenöffnung eingesetzt. An der oberen bzw. Distalseite (6) der Scheibenklappe (3) ist eine Ringfläche (9) mit einem krummlinigen Profil vorgesehen, durch welche eine Herabsetzung der Masse der Scheibenklappe (3) erreicht wird. Die Scheibenklappe (3) ist zwischen Hubbegrenzern (4, 5) schwimmend eingesetzt.

Fig. 1

1/1

FIG.1

FIG.2

## INTERNATIONAL SEARCH REPORT

International Application No PCT/SU 88/00137

**I. CLASSIFICATION OF SUBJECT MATTER** (if several classification symbols apply, indicate all) [6]

According to International Patent Classification (IPC) or to both National Classification and IPC

IPC[4]      A 61 F 2/24

**II. FIELDS SEARCHED**

| Minimum Documentation Searched [7] | |
|---|---|
| Classification System | Classification Symbols |
| IPC[4] | A 61 F 2/24 |

Documentation Searched other than Minimum Documentation·
to the Extent that such Documents are Included in the Fields Searched [8]

**III. DOCUMENTS CONSIDERED TO BE RELEVANT [9]**

| Category [*] | Citation of Document, [11] with indication, where appropriate, of the relevant passages [12] | Relevant to Claim No. [13] |
|---|---|---|
| A | DE, B2, 2640246, (Shiley Inc., Irvine), 25 June 1981 (25.06.81), see the claims figures 3,5 & US, A, 4057857 | 1 |
| A | US, A, 4416029, (Robert L.Kaster), 22 November 1983 (22.11.83), see the claims, figure 1, cited in the description | 1 |
| A,P | US, A, 4713071, (Naum A. Iofis et al.), 15 December 1987 (15.12.87), see the claims, figures 1,5 cited in the description | 1 |

\* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 31 August 1988 (31.08.88) | 5 October 1988 (05.10.88) |
| International Searching Authority | Signature of Authorized Officer |
| ISA/SU | |